# EUROPEAN PATENT APPLICATION

(11) **EP 4 074 712 A1**
(43) Date of publication of application: **19.10.2022**
(21) Application number: 21168106.9
(22) Date of filing: 13.04.2021
(51) Int. Cl.: C07D 471/04

(54) **PROCESS FOR THE SYNTHESIS OF 1,3-DIHYDRO-IMIDAZO[4,5-B]PYRIDIN-2-ONE AND/OR DERIVATIVES THEREOF**

(71) Applicant: Siegfried AG, 4800 Zofingen (CH)
(72) Inventor: WEBER, Beat Theodor, 4800 Zofingen (CH); HUANG, Fuping, Nantong, 226010 (CN); CHENG, Mounuo, Nantong, 226010 (CN); CAO, Chunli, Nantong, 226010 (CN)
(74) Representative: Isarpatent

(57) **Abstract**

The present invention relates to a novel method of producing a compound of Formula (I) from a compound of formula (II) by a novel cyclisation process, as well as a method of producing an acid adduct of the compound of Formula (I) 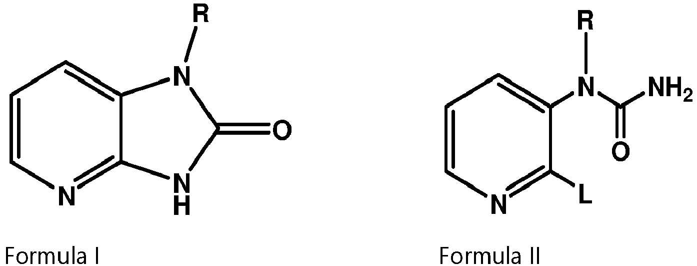 wherein L represents a leaving group, and R represents hydrogen, a substituted or unsubstituted linear, branched and/or cyclic alkyl group that may contain one or more hetero atoms in the linear, branched and/or cyclic alkyl chain, a substituted or unsubstituted aromatic or heteroaromatic group, a substituted or unsubstituted linear, branched and/or cyclic aralkyl or heteroaromatic alkyl group that may contain one or more hetero atoms in the linear, branched and/or cyclic alkanediyl chain, or a substituted or unsubstituted alkylaryl or alkyl heteroaromatic group with at least one linear, branched and/or cyclic alkyl residue that may contain one or more hetero atoms in the linear, branched and/or cyclic alkyl chain.

## Description

### Field of the invention

The present invention relates to a method of producing a compound of Formula (I), particularly 1,3-dihydro-imidazo[4,5-b]pyridin-2-one derivatives. Particularly, the present invention relates to a novel method of producing derivatives of 3-(piperidin-4-yl)-1H-imidazo[4,5-b]pyridin-2(3H)-one, particularly a compound of Formula (1a) from a compound of formula (IIa), by a novel cyclisation process, as well as a method of producing an acid adduct of the compound of Formula (I) 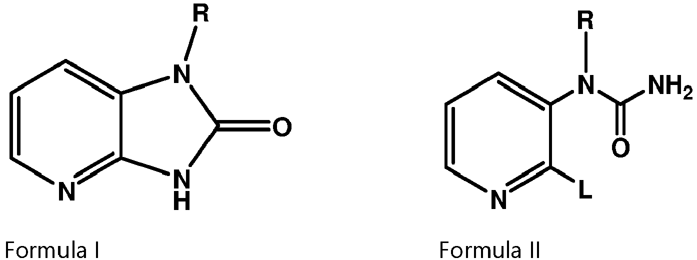 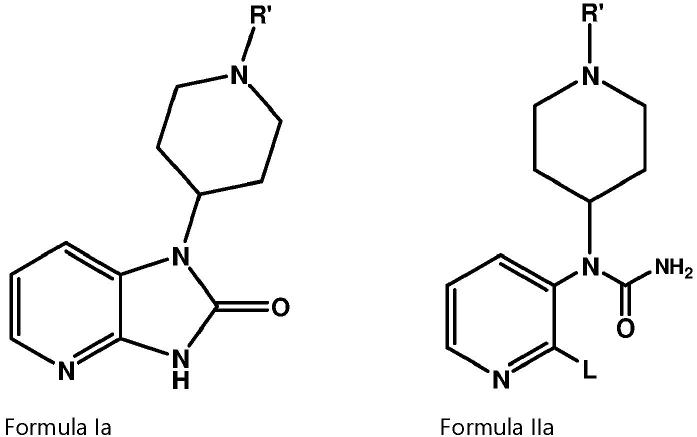 wherein L represents a leaving group, R represents hydrogen, a substituted or unsubstituted linear, branched and/or cyclic alkyl group that may contain one or more hetero atoms in the linear, branched and/or cyclic alkyl chain, a substituted or unsubstituted aromatic or heteroaromatic group, a substituted or unsubstituted linear, branched and/or cyclic aralkyl or heteroaromatic alkyl group that may contain one or more hetero atoms in the linear, branched and/or cyclic alkanediyl chain, or a substituted or unsubstituted alkylaryl or alkyl heteroaromatic group with at least one linear, branched and/or cyclic alkyl residue that may contain one or more hetero atoms in the linear, branched and/or cyclic alkyl chain, and R' represents hydrogen, a substituent, a substituted or unsubstituted linear, branched and/or cyclic alkyl group that may contain one or more hetero atoms in the linear, branched and/or cyclic alkyl chain, a substituted or unsubstituted aromatic or heteroaromatic group, a substituted or unsubstituted linear, branched and/or cyclic aralkyl or heteroaromatic alkyl group that may contain one or more hetero atoms in the linear, branched and/or cyclic alkanediyl chain, or a substituted or unsubstituted alkylaryl or alkyl heteroaromatic group with at least one linear, branched and/or cyclic alkyl residue that may contain one or more hetero atoms in the linear, branched and/or cyclic alkyl chain.

### Background of the Invention

1,3-dihydro-imidazo[4,5-b]pyridin-2-one and its derivatives are precursors of many active pharmaceutical ingredients, already marketed or still under development. Therapeutic areas for those substances of interest are as wide as the treatment of cancer, erectile dysfunction, diabetes, migraine, the use as antithrombotic, as analgesic and others. Namely telcagepant, rimegepant, imigliptin ((R)-2-((7-(3-aminopiperidin-1-yl)-3,5-dimethyl-2-oxo-2,3-dihydro-1H-imidazo[4,5-b]pyridin-1-yl)methyl)benzonitrile), FR-238831 (1-(3-chloro-4-methoxybenzyl)-3-(4-hydroxycyclohexyl)-2-oxo-2,3-dihydro-1H-imidazo[4,5-b]pyridine-6-carbonitrile), CJS-3678 (1-(4-chlorophenyl)-3-(3-(2-oxo-2,3-dihydro-1H-imidazo[4,5-b]pyridin-7-yloxy)phenyl)urea), CJS-3255 (7-(4-(1-(4-chloro-3-(trifluoromethyl)phenylamino)vinylamino)phenoxy)-3-methyl-1H-imidazo[4,5-b]pyridin-2(3H)-one), or AA-012 (1-(3-tert-butyl-1-phenyl-1H-pyrazol-5-yl)-3-(4-(1-methyl-2-oxo-2,3-dihydro-1H-imidazo[4,5-b]pyridin-7-yloxy)naphthalen-1-yl)urea) and various other active pharmaceutical ingredients, mainly out of the group of CGRP (Calcitonin gene-related peptide) receptor antagonists are examples of active pharmaceutical ingredients having 1,3-dihydro-imidazo[4,5-b]pyridin-2-one or one of its derivatives as a substructure.

Rimegepant and imigliptin are examples of substances where in their synthesis 1,3-dihydro-imidazo[4,5-b]pyridin-2-one or its derivatives are key precursors. Rimegepant is an active pharmaceutical compound used to treat migraine in adults, whereas imigliptine is used to treat diabetes.

There is an increasing demand of having access to ecologically and economically accessible precursors and suitable manufacturing processes.

### State of the art

Few industrially applicable manufacturing processes of 1,3-dihydro-imidazo[4,5-b]pyridin-2-one are available.

The manufacturing process with carboyldiimidazol (CDI) is widely used in the pharmaceutical industry and disclosed in different patents and patent applications. For example, in AU 2009/278442, FR 2 605 008, WO 2013/130890, and WO 2015/065336 2,3-diaminopyridine derivatives are used as the starting material. CH 635 586 discloses a synthesis where COCl₂ is used instead of CDl.

Leahy K. D. et al., Organic Process Research & Development, 2012, Vol. 16, Issue 2, pp 244-249, disclose a synthetic route starting from tert-butyl 4-(2-aminopyridin-3-ylamino)-piperidine-1-carboxylate to *tert-*butyl 4-(2-oxo-2,3-dihydro-1H-imidazo[4,5-b]pyridin-1-yl)-piperidine-1-carboxylate. Authors use carboyldiimidazol (CDI) as key agent therein. US 4,144,341 describes a cyclization process of a different molecule requiring elevated temperatures and resulting in a yield of 10.3 %.

However, it is desirable to establish a sustainable route of synthesis working under mild reaction conditions, using less toxic and more environmental friendly agents, that are easily accessible and available.

Thus, an object of the present invention is presenting a method for the production of a compound of Formula (I), e.g. 1,3-dihydro-imidazo[4,5-b]pyridin-2-one or a derivative thereof, as precursor of various pharmaceutical compounds.

### Summary of the invention

The present invention offers a novel method for the synthesis of a compound of Formula (I), wherein R represents hydrogen, a substituted or unsubstituted linear, branched and/or cyclic alkyl group that may contain one or more hetero atoms in the linear, branched and/or cyclic alkyl chain, a substituted or unsubstituted aromatic or heteroaromatic group, a substituted or unsubstituted linear, branched and/or cyclic aralkyl or heteroaromatic alkyl group that may contain one or more hetero atoms in the linear, branched and/or cyclic alkanediyl chain, or a substituted or unsubstituted alkylaryl or alkyl heteroaromatic group with at least one linear, branched and/or cyclic alkyl residue that may contain one or more hetero atoms in the linear, branched and/or cyclic alkyl chain.

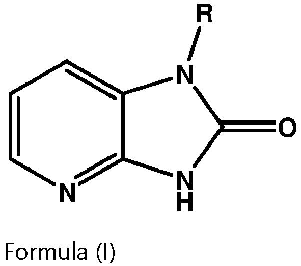

Inventors found a method of such a novel syntheses using easily available agents having an ecological and an economical advantage to the manufacturing processes used today. The innovative process offers high yield under mild reaction conditions. Particularly, according to certain embodiments, a low boiling of the solvents found to be suitable in this invention allows easy recycling and environmental friendly closed circuits for solvents. The disclosed invention avoids complex or complex to handle catalysts, especially expensive metal catalysts based on palladium. This novel method also reduces the amount of produced side products and impurities caused by the used agents in the final product that are difficult to remove.

In a first aspect, the present invention relates to a method of producing a compound of Formula (I) from a compound of Formula (II), comprising:
reacting the compound of Formula (II) in a solvent or a solvent mixture, in the presence of a Cu catalyst,
a diamine additive, and
optionally a base, particularly an inorganic base
to form the compound of Formula (I) 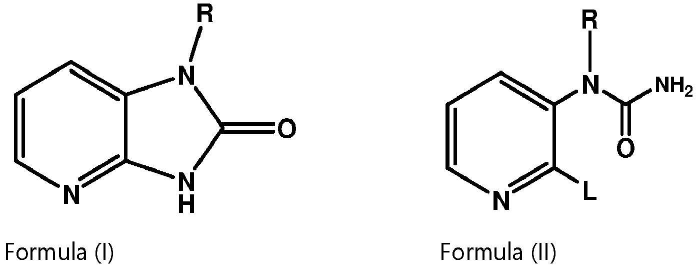 wherein L represents a leaving group, and R represents hydrogen, a substituted or unsubstituted linear, branched and/or cyclic alkyl group that may contain one or more hetero atoms in the linear, branched and/or cyclic alkyl chain, a substituted or unsubstituted aromatic or heteroaromatic group, a substituted or unsubstituted linear, branched and/or cyclic aralkyl or heteroaromatic alkyl group that may contain one or more hetero atoms in the linear, branched and/or cyclic alkanediyl chain, or a substituted or unsubstituted alkylaryl or alkyl heteroaromatic group with at least one linear, branched and/or cyclic alkyl residue that may contain one or more hetero atoms in the linear, branched and/or cyclic alkyl chain.

Further disclosed is a method of producing an acid adduct of a compound of Formula (I) from a compound of Formula (II), comprising:
- reacting the compound of Formula (II) in a solvent or a solvent mixture in the presence of a Cu catalyst,
   a diamine additive, and
   optionally a base, particularly an inorganic base,
   to form the compound of Formula (I), 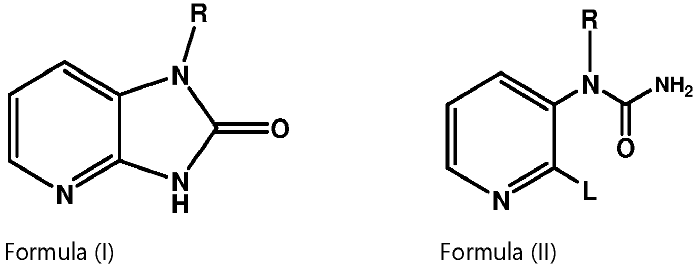 wherein L represents a leaving group, and R represents hydrogen, a substituted or unsubstituted linear, branched and/or cyclic alkyl group that may contain one or more hetero atoms in the linear, branched and/or cyclic alkyl chain, a substituted or unsubstituted aromatic or heteroaromatic group, a substituted or unsubstituted linear, branched and/or cyclic aralkyl or heteroaromatic alkyl group that may contain one or more hetero atoms in the linear, branched and/or cyclic alkanediyl chain, or a substituted or unsubstituted alkylaryl or alkyl heteroaromatic group with at least one linear, branched and/or cyclic alkyl residue that may contain one or more hetero atoms in the linear, branched and/or cyclic alkyl chain, and
- reacting the compound of Formula (I) with an acid.

Further aspects and embodiments of the invention are disclosed in the dependent claims and can be taken from the following description, figures and examples, without being limited thereto.

### Detailed description of the present invention

### Definitions

Unless defined otherwise, technical and scientific terms used herein have the same meaning as commonly understood by one of ordinary skill in the art to which this invention belongs.

Amounts within the present invention are given in wt.%, unless stated otherwise or clear from context.

Within the scope of the disclosure, a copper catalyst / Cu catalyst is not particularly restricted and encompasses Cu itself as well as compounds thereof, e.g. Cu salts and Cu complexes, but preferably refers to Cu salts, particularly Cu(I) and/or Cu(II) salts.

In structural formulae, Bn is a benzyl group, BOC is a *tert*-butyloxycarbonyl group.

Before the invention is described in exemplary detail, it is to be understood that this invention is not limited to the particular component parts of the process steps of the methods described herein as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise. It is moreover to be understood that, in case parameter ranges are given which are delimited by numeric values, the ranges are deemed to include these limitation values.

In a first aspect, the present invention relates to a method of producing a compound of Formula (I) from a compound of Formula (II), comprising:
reacting the compound of Formula (II) in a solvent or a solvent mixture, in the presence of a Cu catalyst,
a diamine additive, and
optionally a base, particularly an inorganic base
to form the compound of Formula (I) 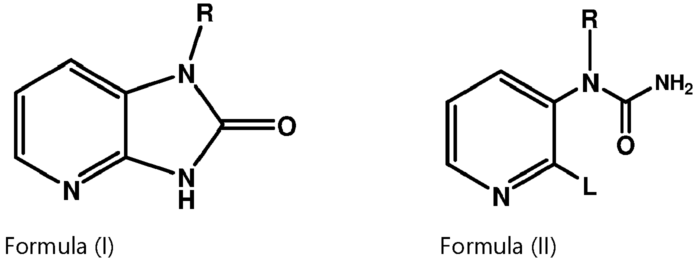 wherein L represents a leaving group, and R represents hydrogen, a substituted or unsubstituted linear, branched and/or cyclic alkyl group that may contain one or more hetero atoms in the linear, branched and/or cyclic alkyl chain, a substituted or unsubstituted aromatic or heteroaromatic group, a substituted or unsubstituted linear, branched and/or cyclic aralkyl or heteroaromatic alkyl group that may contain one or more hetero atoms in the linear, branched and/or cyclic alkanediyl chain, or a substituted or unsubstituted alkylaryl or alkyl heteroaromatic group with at least one linear, branched and/or cyclic alkyl residue that may contain one or more hetero atoms in the linear, branched and/or cyclic alkyl chain.

In the present method, the cyclization of the compound of Formula (II) to the compound of Formula (I), e.g. the cyclization of specific 1-(pyridin-3-yl)urea compounds to particular 1H-imidazo[4,5-b]pyridin-2(3H) compounds, generally can be described with the following reaction scheme 1: 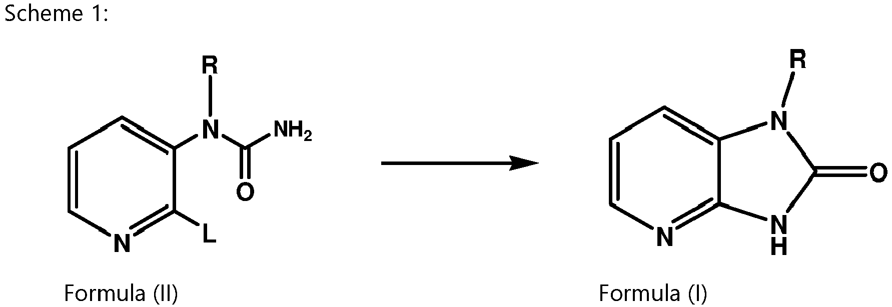 wherein
L represents a leaving group, and
R represents hydrogen, a substituted or unsubstituted linear, branched and/or cyclic alkyl group that may contain one or more hetero atoms in the linear, branched and/or cyclic alkyl chain, a substituted or unsubstituted aromatic or heteroaromatic group, a substituted or unsubstituted linear, branched and/or cyclic aralkyl or heteroaromatic alkyl group that may contain one or more hetero atoms in the linear, branched and/or cyclic alkanediyl chain, or a substituted or unsubstituted alkylaryl or alkyl heteroaromatic group with at least one linear, branched and/or cyclic alkyl residue that may contain one or more hetero atoms in the linear, branched and/or cyclic alkyl chain.

The present invention thus particularly relates to a method of producing 1,3-dihydro-imidazo[4,5-b]pyridin-2-one, or derivatives thereof, starting from a 1-(pyridin-3-yl)urea compound, i.e. 1-(pyridin-3-yl)urea or a derivative thereof, preferably 1-(2-halopyridin-3-yl)urea or a derivative thereof, in presence of a copper catalyst, particularly a copper (I) or copper (II) compound, particularly a copper (I) or copper (II) salt, as catalyst, and further in presence of a diamine additive, and optionally in presence of a base, particularly an inorganic base.

In the compound of Formula (II) the leaving group L is not particularly restricted and can be any chemical group capable of acting as leaving group. Any suitable leaving group can be used, e.g. a nitro group, an ester group of alkyl and/or aryl sulfonic acids like mesyl, tosyl, an ester group of alkyl and/or aryl carboxylic acids, halogen, a pseudohalogen group chosen from -CN, -N₃, -OCN, -NCO, -CNO, -SCN, -NCS, -SeCN, etc. According to certain embodiments, the leaving group L is a halogen, particularly F, Cl, Br, or I, e.g. Cl or Br. According to certain embodiments, the leaving group L is chloride.

In the compound of Formula (II), and thus also in the compound of Formula (I), the group R represents hydrogen,
a substituted or unsubstituted linear, branched and/or cyclic alkyl group that may contain one or more hetero atoms in the linear, branched and/or cyclic alkyl chain, preferably having 1 to 40 C atoms, particularly having 1 to 20 C atoms,
a substituted or unsubstituted aromatic group, preferably having 6 to 40 C atoms, particularly having 6 to 20 C atoms,
a substituted or unsubstituted heteroaromatic group, preferably having 2 to 40 C atoms, particularly having 3 to 20 C atoms, wherein the heteroatom of the heteroaromatic group particularly is N,
a substituted or unsubstituted linear, branched and/or cyclic aralkyl group (with an aryl group bonded to a linear, branched and/or cyclic alkanediyl chain) that may contain one or more hetero atoms in the linear, branched and/or cyclic alkanediyl chain, preferably having 7 to 40 C atoms, particularly having 7 to 20 C atoms,
a substituted or unsubstituted linear, branched and/or cyclic heteroaromatic alkyl group (with a heteroaromatic group to a linear, branched and/or cyclic alkanediyl chain) that may contain one or more hetero atoms in the linear, branched and/or cyclic alkanediyl chain, preferably having 3 to 40 C atoms, particularly having 4 to 20 C atoms, wherein the heteroatom of the heteroaromatic group particularly is N,
a substituted or unsubstituted alkylaryl group with at least one linear, branched and/or cyclic alkyl residue that may contain one or more hetero atoms in the linear, branched and/or cyclic alkyl chain, preferably having 7 to 40 C atoms, particularly having 7 to 20 C atoms, or
a substituted or unsubstituted alkylheteroaryl group with at least one linear, branched and/or cyclic alkyl residue that may contain one or more hetero atoms in the linear, branched and/or cyclic alkyl chain, preferably having 7 to 40 C atoms, particularly having 7 to 20 C atoms, wherein the heteroatom of the heteroaromatic group particularly is N.

R groups of particular interest in the scope of this application are moieties that represent either the full structure of Active Pharmaceutical Ingredients in question, particularly telcagepant, rimegepant, imigliptin ((R)-2-((7-(3-aminopiperidin-1-yl)-3,5-dimethyl-2-oxo-2,3-dihydro-1H-imidazo[4,5-b]pyridin-1-yl)methyl)benzonitrile), FR-238831 (1-(3-chloro-4-methoxybenzyl)-3-(4-hydroxycyclohexyl)-2-oxo-2,3-dihydro-1H-imidazo[4,5-b] pyridine-6-carbonitrile), CJS-3678 (1-(4-chlorophenyl)-3-(3-(2-oxo-2,3-dihydro-1H-imidazo[4,5-b]pyridin-7-yloxy)phenyl)urea), CJS-3255 (7-(4-(1-(4-chloro-3-(trifluoromethyl)phenyl-amino)vinylamino)phenoxy)-3-methyl-1H-imidazo[4,5-b]pyridin-2(3H)-one), or AA-012 (1-(3-tert-butyl-1-phenyl-1H-pyrazol-5-yl)-3-(4-(1-methyl-2-oxo-2,3-dihydro-1H-imidazo[4,5-b]pyridin-7-yloxy)naphthalen-1-yl)urea), or precursors that allow the construction of said products. Examples are substituted or unsubstituted linear and/or branched alkyl groups, cycloalky groups, aryl groups, heteroaryl groups or groups with a mix of these elements. The group may contain one or several heteroatoms from the group of N, P, O, S, halogens (e.g. F, Cl, Br, and/or I). The R group is particularly characterized by the fact that it remains unchanged during the transformation in the present method.

In the residues R having one or more hetero atoms in the linear, branched and/or cyclic alkyl chain, the heteroatom in the linear, branched and/or cyclic alkyl chain is not particularly restricted, and preferably is at least one of N, O, S, Se, and/or P, and particularly is N.

In the residue R, as well as in the residue R' discussed herein, the substituent - if present - is not particularly restricted. Particularly, according to certain embodiments, the substituent is chosen from:
- alkoxy carbonyl groups, alkenoxy carbonyl groups, and aralkyloxy carbonyl groups -(CO)-OR¹, particularly linear, branched and/or cyclic alkoxy carbonyl groups having 1 to 10, particularly 1 to 4 C atoms (i.e. R¹ being a linear, branched and/or cyclic alkyl residue with 1 to 10, particularly 1 to 4 C atoms), e.g. a methoxycarbonyl group, ethoxycarbonyl group, propoxycarbonyl group, butoxycarbonyl group, tert-butyloxycarbonyl group, etc.; linear, branched and/or cyclic alkenoxy carbonyl groups having 2 to 10, particularly 2 to 4 C atoms (i.e. R¹ being a linear, branched and/or cyclic alkenyl residue with 2 to 10, particularly 2 to 4 C atoms) , e.g. an allyloxycarbonyl group, etc.; aryloxy carbonyl groups having 6 to 40, particularly 6 to 20 C atoms (i.e. R¹ being an aromatic residue having 6 to 40, particularly 6 to 20 C atoms), or linear, branched and/or cyclic aralkyloxy carbonyl groups having 7 to 40, particularly 7 to 20 C atoms (i.e. R¹ being a linear, branched and/or cyclic aralkyl residue with 7 to 40, particularly 7 to 20 C atoms), e.g.a benzyloxy carbonyl group, fluorenmethyloxy carbonyl group, etc., further particularly linear, branched and/or cyclic alkoxy carbonyl groups having 1 to 10, particularly 1 to 4 C atoms;
- alkyl carbonyl oxy groups -O(CO)-R², particularly linear, branched and/or cyclic alkyl carbonyl oxy groups having 1 to 10, particularly 1 to 4 C atoms (i.e. R² being a linear, branched and/or cyclic alkyl residue with 1 to 10, particularly 1 to 4 C atoms), arylcarbonyl oxy groups having 6 to 40, particularly 6 to 20 C atoms (i.e. R² being an aromatic residue having 6 to 40, particularly 6 to 20 C atoms), or linear, branched and/or cyclic aralkylcarbonyloxy groups having 7 to 40, particularly 7 to 20 C atoms (i.e. R² being a linear, branched and/or cyclic aralkyl residue with 7 to 40, particularly 7 to 20 C atoms);
- alkoxy groups -OR³, particularly linear, branched and/or cyclic alkoxy groups having 1 to 10, particularly 1 to 4 C atoms (i.e. R³ being a linear, branched and/or cyclic alkyl residue with 1 to 10, particularly 1 to 4 C atoms), e.g. a methoxy group, ethoxy group, propoxy group, butoxy group, tert-butoxy group, aryloxy groups having 6 to 40, particularly 6 to 20 C atoms (i.e. R³ being an aromatic residue having 6 to 40, particularly 6 to 20 C atoms), or linear, branched and/or cyclic aralkyloxy groups having 7 to 40, particularly 7 to 20 C atoms(i.e. R³ being a linear, branched and/or cyclic aralkyl residue with 7 to 40, particularly 7 to 20 C atoms);
- alkyl amide group -(CO)NR⁴R⁵, particularly linear, branched and/or cyclic alkyl amide groups having 1 to 10, particularly 1 to 4 C atoms, wherein R⁴ and R⁵ can be the same or different, with the provisio that at least one of R⁴ and R⁵ is not H;
- sulfone groups -(SO₂)-R⁶, particularly wherein R⁶ is chosen from H, linear, branched and/or cyclic alkyl residues with 1 to 10, particularly 1 to 4 C atoms, aryl groups having 6 to 40, particularly 6 to 20 C atoms, or linear, branched and/or cyclic aralkyl groups having 7 to 40, particularly 7 to 20 C atoms;
- sulfoxide groups -(SO)-R⁷, particularly wherein R⁷ is chosen from H, linear, branched and/or cyclic alkyl residues with 1 to 10, particularly 1 to 4 C atoms, aryl groups having 6 to 40, particularly 6 to 20 C atoms, or linear, branched and/or cyclic aralkyl groups having 7 to 40, particularly 7 to 20 C atoms;
- thioether groups -SR⁸, particularly wherein R⁸ is chosen from H, linear, branched and/or cyclic alkyl residues with 1 to 10, particularly 1 to 4 C atoms, aryl groups having 6 to 40, particularly 6 to 20 C atoms, or linear, branched and/or cyclic aralkyl groups having 7 to 40, particularly 7 to 20 C atoms;
- carboxamide groups -(CO)-NR⁹R¹⁰, particularly wherein R⁹ and R¹⁰ are chosen from H, linear, branched and/or cyclic alkyl residues with 1 to 10, particularly 1 to 4 C atoms, aryl groups having 6 to 40, particularly 6 to 20 C atoms, and/or linear, branched and/or cyclic aralkyl groups having 7 to 40, particularly 7 to 20 C atoms, wherein R⁹ and R¹⁰ can be the same or different,
- carboxylic acid groups -COOR¹¹, particularly wherein R¹¹ is chosen from H, linear, branched and/or cyclic alkyl residues with 1 to 10, particularly 1 to 4 C atoms, aryl groups having 6 to 40, particularly 6 to 20 C atoms, or linear, branched and/or cyclic aralkyl groups having 7 to 40, particularly 7 to 20 C atoms,
- halogens, e.g. F, Cl, Br, I, particularly Cl and/or Br, more particularly Cl,
- NH₂, or
- OH.

According to certain embodiments, the substituent is a tert-butyloxycarbonyl (Boc) group.

According to certain embodiments, the group R in the compound of Formula (II), and thus also in the compound of Formula (I), represents hydrogen.

According to certain embodiments, the group R in the compound of Formula (II), and thus also in the compound of Formula (I), represents a substituted or unsubstituted linear, branched and/or cyclic alkyl group that may contain one or more hetero atoms in the linear, branched and/or cyclic alkyl chain, preferably having 1 to 40 C atoms, particularly having 1 to 20 C atoms, e.g. a substituted or unsubstituted methyl, ethyl, propyl, isopropyl, cyclopropyl, butyl, isobutyl, tertbuyl, cyclobutyl, cyclohexyl, piperidin-4-yl, 1-(*tert*-butoxycarbonyl)-piperidin-4-yl (1-Boc-piperidin-4-yl), etc., group. According to certain embodiments, the group R in the compound of Formula (II), and thus also in the compound of Formula (I), represents a 1-substituted or a unsubstituted piperidin-4-yl group, particularly a 1*-*(*tert-*butoxycarbonyl)piperidin-4-yl group.

According to certain embodiments, the group R in the compound of Formula (II), and thus also in the compound of Formula (I), represents a substituted or unsubstituted aromatic group, preferably having 6 to 40 C atoms, particularly having 6 to 20 C atoms, e.g. a substituted or unsubstituted phenyl or naphthyl group.

According to certain embodiments, the group R in the compound of Formula (II), and thus also in the compound of Formula (I), represents a substituted or unsubstituted linear, branched and/or cyclic aralkyl group that may contain one or more hetero atoms in the linear, branched and/or cyclic alkanediyl chain, preferably having 7 to 40 C atoms, particularly having 7 to 20 C atoms, e.g. a benzyl, phenethyl, phenylpropyl, naphthylmethyl, 1-benzylpiperidin-4-yl, group, etc. According to certain embodiments, the group R in the compound of Formula (II), and thus also in the compound of Formula (I), represents a substituted or unsubstituted 1-benzylpiperidin-4-yl group, particularly an unsubstituted 1-benzylpiperidin-4-yl group.

According to certain embodiments, the group R in the compound of Formula (II), and thus also in the compound of Formula (I), represents a substituted or unsubstituted alkylaryl group that may contain one or more hetero atoms in the linear, branched and/or cyclic alkyl chain, preferably having 7 to 40 C atoms, particularly having 7 to 20 C atoms, e.g. a substituted or unsubstituted methylphenyl, ethylphenyl, propylphenyl, isopropylphenyl, etc. group.

According to certain embodiments, R represents hydrogen, or a substituted or unsubstituted linear, branched and/or cyclic alkyl group or aralkyl group with 1 to 12 carbons that may contain none, one or more hetero atoms. Preferably R is a piperidine group - particularly a piperidin-4-yl group, a N-substituted piperidine derivative - particularly a N-substituted piperidin-4-yl group, a 1-benzylpiperidin-4-yl group, or benzyl. Preferably R is piperidine, particularly a piperidin-4-yl group, N-substituted (1-substituted) by tert-butyloxycarbonyl, or is a 1-benzylpiperidin-4-yl group.

A schematic for a preferred reaction of a compound of Formula (IIa) to a compound of Formula (Ia) in a preferred method is shown in the following scheme 2. 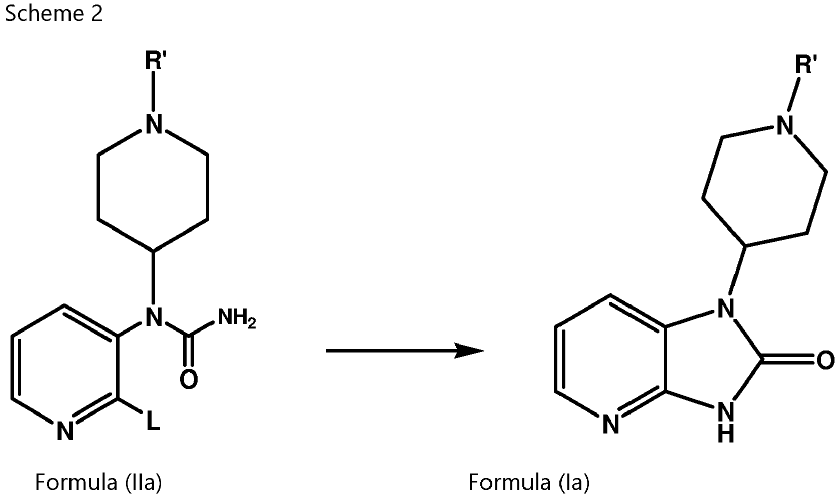
wherein L represents a leaving group, and
R' represents hydrogen, a substituent as defined above, a substituted or unsubstituted linear, branched and/or cyclic alkyl group that may contain one or more hetero atoms in the linear, branched and/or cyclic alkyl chain, a substituted or unsubstituted aromatic or heteroaromatic group, a substituted or unsubstituted linear, branched and/or cyclic aralkyl or heteroaromatic alkyl group that may contain one or more hetero atoms in the linear, branched and/or cyclic alkanediyl chain, or a substituted or unsubstituted alkylaryl or alkyl heteroaromatic group with at least one linear, branched and/or cyclic alkyl residue that may contain one or more hetero atoms in the linear, branched and/or cyclic alkyl chain,
particularly where R' represents
hydrogen,
a substituent as defined above,
a substituted or unsubstituted linear, branched and/or cyclic alkyl group that may contain one or more hetero atoms in the linear, branched and/or cyclic alkyl chain having 1 to 35 C atoms, preferably 1 to 15 C atoms, particularly 1 to 4 C atoms,
a substituted or unsubstituted aromatic group with 6 to 35 C atoms, preferably 6 to 20 C atoms, particularly 6 to 12 C atoms,
a substituted or unsubstituted heteroaromatic group having 2 to 35 C atoms, preferably 3 to 15 C atoms, particularly 3 to 12 C atoms wherein the heteroatom of the heteroaromatic group particularly is N,
a substituted or unsubstituted linear, branched and/or cyclic aralkyl group that may contain one or more hetero atoms in the linear, branched and/or cyclic alkanediyl chain having 7 to 35 C atoms, preferably 7 to 15 C atoms, particularly 7 to 12 C atoms,
a substituted or unsubstituted linear, branched and/or cyclic heteroaromatic alkyl group that may contain one or more hetero atoms in the linear, branched and/or cyclic alkanediyl chain,, preferably having 3 to 35 C atoms, particularly having 4 to 15 C atoms, wherein the heteroatom of the heteroaromatic group particularly is N,
a substituted or unsubstituted alkyl aryl group with at least one linear, branched and/or cyclic alkyl residue that may contain one or more hetero atoms in the alkyl chain having 7 to 35 C atoms, preferably 7 to 15 C atoms, particularly 7 to 12 C atoms, or
a substituted or unsubstituted alkylheteroaryl group with at least one linear, branched and/or cyclic alkyl residue that may contain one or more hetero atoms in the linear, branched and/or cyclic alkyl chain having 7 to 35 C atoms, preferably 7 to 15 C atoms, particularly having 7 to 20 C atoms, wherein the heteroatom of the heteroaromatic group particularly is N
with the substituents as defined above; and particularly represents either a benzyl group or a *tert*-butyloxycarbonyl group.

In the present method, the Cu catalyst is not particularly restricted. Particularly, the origin of the copper catalyst is not particularly restricted.

According to certain embodiments, as copper catalyst any salt of copper(I) or copper(II) can be used. Preferably the catalyst is a copper (I) and/or a copper (II) salt having the formulae CuX, Cu₂Y, CuX₂ or CuY, wherein X is halogen or any other monovalent anion, preferably halogen or OAc (Ac being an acetyl group (CO)CH₃), and wherein Y is a divalent anion, both not particularly limited. Preferably X is halogen, particularly Cl, Br, or I, e.g. Cl or I; or OAc; and Y is sulfate. According to certain embodiments, the Cu catalyst is Cul, CuCl, CuBr, Cu(OAc)₂, and/or CuSO₄, particularly CuCl, CuBr, Cul, and/or CuSO₄. According to certain embodiments, the Cu catalyst is CuCl, CuBr, Cul, Cu(OAc)₂, or CuSO₄, particularly CuCl, CuBr, Cul, or CuSO₄, e.g. CuCl, Cul, or CuSO₄.

The amount of the copper catalyst is not particularly limited. According to certain embodiments, the copper catalyst is added in amounts between and including 0.1 equivalents and 0.7 equivalents relative to the compound of Formula (II) - respectively the compound of Formula (IIa), preferably in an amount between and including 0.1 equivalents and 0.5 equivalents.

The present method is carried in the presence of a diamine additive. The nature of the diamine additive is not particularly restricted. It can be e.g. N,N'-dimethyl-ethylene diamine (DMEDA), ethylene diamine (EDA), propylene diamine (PDA), butylene diamine, pentylene diamine, 1,2-diaminocyclopentane, trans-1,2-diaminocyclohexane (DACH), phenylenediamine, e.g. o-phenylenediamine or diaminotoluene, e.g. 2,3-diaminotoluene, 3,4-diaminotoluene, or diaminonaphthalene, e.g. 1,8-diaminonaphthalene, or 1,10-phenanthroline or mixtures thereof. According to certain embodiments, the diamine additive is a bidentate ligand, particularly in which both nitrogen atoms allow binding to the copper of the catalyst. Without being bound to any theory, it is assumed that a bidentate ligand enables the cyclization reaction due to aiding in effective ring formation.

According to certain embodiments, the diamine additive is chosen from the group consisting of N,N'-dimethyl-ethylene diamine (DMEDA), ethylene diamine (EDA), propylene diamine (PDA), or trans-1,2-diaminocyclohexane (DACH), or mixtures thereof. According to preferred embodiments, the diamine additive is chosen from EDA, PDA and DACH, further preferably EDA and PDA, and particularly is ethylene diamine (EDA).

The amount of the diamine additive is between and including 0.1 equivalents and 9.0 equivalents, preferably between and including 0.2 and 9.0 equivalents, further preferably between and including 0.25 equivalents and 7.5 equivalents, in relation to the compound of Formula (II) - respectively to the compound of Formula (IIa). According to certain embodiments, the equivalents of diamine additive is equal or greater than the equivalents of the of the copper catalyst. The diamine additive is preferably added with less than 9.0 equivalents, in relation to the compound of Formula (II) - respectively the compound of Formula (IIa).

If a base is added in the present method, it is not particularly restricted. According to certain embodiments, a base is added, particularly an inorganic base. According to certain embodiments, the process is carried in the presence of an inorganic base, which nature is not particularly restricted. According to certain embodiments, the base is a salt of an alkaline earth metal and/or an alkali metal, e.g. a salt of an alkaline earth metal or an alkali metal.

According to certain embodiments, the base is a salt selected from, but not limited to, the group of carbonates or bicarbonates, preferably from the group potassium carbonate, potassium bicarbonate, sodium carbonate and sodium bicarbonate, further preferably potassium carbonate and sodium carbonate.

The amount of the base in the reaction is not particularly limited. Suitable amounts of the inorganic base are, according to certain embodiments, between and including 0 equivalents and 5.0 equivalents in relation to the compound of Formula (II) - respectively the compound of Formula (IIa), e.g. between and including 2.0 equivalents and 5.0 equivalents, preferably less or equal to 3.5 equivalents, e.g. less or equal to 3.0 equivalents, more preferably equal to 2.0 equivalents or more and equal to 3.0 equivalents or less.

In the present method the solvent or solvent mixture is not particularly restricted. The solvent may comprise an organic solvent, e.g. at least an alcohol, a ketone, an ether, an amide, a sulfone, a sulfoxide, and/or a hydrocarbon, e.g. an aromatic hydrocarbon; e.g. an ether, an amide, a sulfone, a sulfoxide, and/or a hydrocarbon, e.g. an aromatic hydrocarbon, either as sole solvent, or mixtures thereof. The solvent mixture may contain any organic solvent, e.g. at least an alcohol, a ketone, an ether, an amide, a sulfone, a sulfoxide, a hydrocarbon, e.g. an aromatic hydrocarbon, etc.; e.g. an ether, an amide, a sulfone, a sulfoxide, a hydrocarbon, e.g. an aromatic hydrocarbon, or mixtures of more of these with water.

According to certain embodiments, the solvent or solvent mixture contains at least a polar solvent, particularly water; an alcohol like methanol, ethanol, propanol, butanol, etc.; an amide like dimethylformamide (DMF) or dimethylacetamide; a sulfone like sulfolane; a sulfoxide like dimtehylsulfoxide; etc. If a solvent mixture is used in the present method, it is preferred to use a mixture of at least one organic solvent, particularly one organic solvent, e.g. as disclosed above, with water. Preferably a solvent mixture contains water. In such a solvent mixture the amount of water is not particularly restricted.

The ether is not particularly restricted and can be any ether, e.g. dimethyl ether, diethyl ether, methyl ethyl ether, tetrahydrofuran (THF), dioxane (particularly 1,4-dioxane), etc., or mixtures thereof, particularly an ether with a boiling point above 90°C, preferably dioxane, particularly 1,4-dioxane. The amide is not particularly restricted and can be any amide, e.g. formamide, dimethylformamide, acetamide, etc., and can be e.g. dimethylformamide. Also the hydrocarbon is not particularly restricted and can be e.g. a linear, branched, and/or cyclic alkane, and/or an aromatic hydrocarbon like benzene, toluene, ethyl benzene, xylene as mixture of isomers or as pure isomer (o-, m- and/or p-xylene), preferably an aromatic hydrocarbon, particularly toluene. Further, the sulfone is not particularly restricted and can be preferably sulfolane. Also, the sulfoxide is not particularly restricted and can be preferably dimethyl sulfoxide (DMSO).

According to certain embodiment, the cyclisation reaction is carried out in a solvent or solvent mixture allowing at least partial dissolution, e.g. full dissolution of all reagents used in the method. According to certain embodiments, the solvent or solvent mixture allows at least a partial dissolution, e.g. at least 5 wt.%, preferably at least 10 wt.%, more preferably at least 15 wt.% - in relation to the total amount of inorganic base, or even full dissolution of the amount of inorganic base added. For this reason, it is preferred to add a strong polar solvent like water or at least use a solvent like an ether, e.g. dioxane (particularly 1,4-dioxane); an amide, e.g. DMF; a sulfone, e.g. sulfolane; a sulfoxide, e.g. dimethylsulfoxide; a ketone; etc., which can dissolve a salt. In the solvent mixture - containing one or more organic solvents, the amount of water is not particularly restricted, but is preferably 50 vol.% or less and/or 10 vol.% or more with regard to the solvent mixtures.

According to certain embodiments the solvent is an organic solvent containing up to 50 vol.% of water or a mixture of organic solvents containing up to 50 vol.% of water. According to certain embodiments, dioxane (particularly 1,4-dioxane) is used as sole solvent. According to certain embodiments, a mixture of a hydrocarbon and water, particularly an aromatic hydrocarbon and water, especially toluene and water, or a mixture of an ether and water, particularly dioxane (particularly 1,4-dioxane) and water, is used as solvent mixture.

In the present method, the reaction conditions are not particularly limited. For example, in the present method, the reaction temperature is not particularly restricted. According to certain embodiments, it is at least 50°C, at least 70°C, at least 80°C, or even at least 90°C. According to certain embodiments, the reaction is carried out under reflux.

A further, second aspect of the invention relates to a method of producing an acid adduct of a compound of Formula (I) from a compound of Formula (II), comprising:
- reacting the compound of Formula (II) in a solvent or a solvent mixture in the presence of a Cu catalyst,
   a diamine additive, and
   optionally a base, particularly an inorganic base,
   to form the compound of Formula (I), 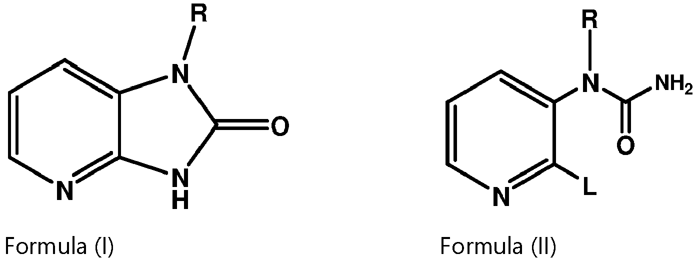 wherein L represents a leaving group, and R represents hydrogen, a substituted or unsubstituted linear, branched and/or cyclic alkyl group that may contain one or more hetero atoms in the linear, branched and/or cyclic alkyl chain, a substituted or unsubstituted aromatic or heteroaromatic group, a substituted or unsubstituted linear, branched and/or cyclic aralkyl or heteroaromatic alkyl group that may contain one or more hetero atoms in the linear, branched and/or cyclic alkanediyl chain, or a substituted or unsubstituted alkylaryl or alkyl heteroaromatic group with at least one linear, branched and/or cyclic alkyl residue that may contain one or more hetero atoms in the linear, branched and/or cyclic alkyl chain, and
- reacting the compound of Formula (I) with an acid.

In this method of the second aspect the first step of reacting the compound of Formula (II) in a solvent or a solvent mixture in the presence of a Cu catalyst, a diamine additive, and optionally a base, particularly an inorganic base, to form the compound of Formula (I) corresponds to the method of the first aspect, so that all embodiments and description with regard to the first aspect can also apply accordingly to this step in the method of the second aspect.

The step of reacting the compound of Formula (I) with an acid in the method of the second aspect is not particularly restricted. Particularly the acid is not restricted and can be an organic or inorganic acid. According to certain embodiments, the acid is an inorganic acid, particularly HCl.

The above embodiments can be combined arbitrarily, if appropriate. Further possible embodiments and implementations of the invention comprise also combinations of features not explicitly mentioned in the foregoing or in the following with regard to the Examples of the invention. Particularly, a person skilled in the art will also add individual aspects as improvements or additions to the respective basic form of the invention.

### Examples

The present invention will now be described in detail with reference to several examples thereof. However, these examples are illustrative and do not limit the scope of the invention.

### General process

General process for manufacturing a 1,3-dihydro-imidazo[4,5-b]pyridin-2-one compound of Formula (III), according to scheme 3, where R' in the particular examples represents either a benzyl group or a *tert*-butyloxycarbonyl group, from a compound of Formula (IV).

The 1-(2-chloropyridin-3-yl)urea compound of Formula (IV) (1.0 eq.) is placed in a flask. A diamine additive, optionally a base, and a copper catalyst are added in suitable amounts. The reaction mixture is heated to a desired temperature, e.g. at reflux or pressure sealed, and kept at this temperature for a suitable time. After completion of the reaction, the reaction mixture is sampled and analyzed.

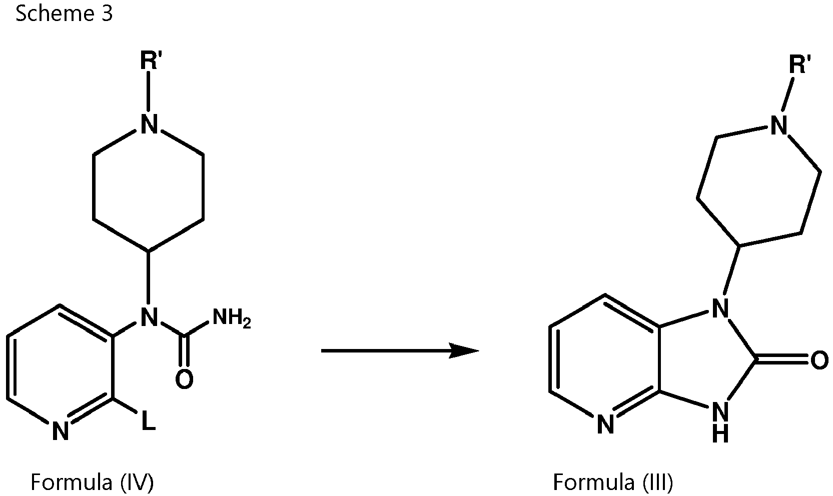

### Reference examples: Preparation of starting materials, i.e. compounds of Formula (IV) with R' being benzyl or Boc

In general, the compounds of Formula (IV) can be prepared from 3-amino-2-chloropiridine using a reductive amination with the respective N-derivative of 4-piperidone, followed by reaction with chlorosulfonylisocyanate (CSI), as indicated in the following schemes 4 and 5 (including the final reaction of the present method).

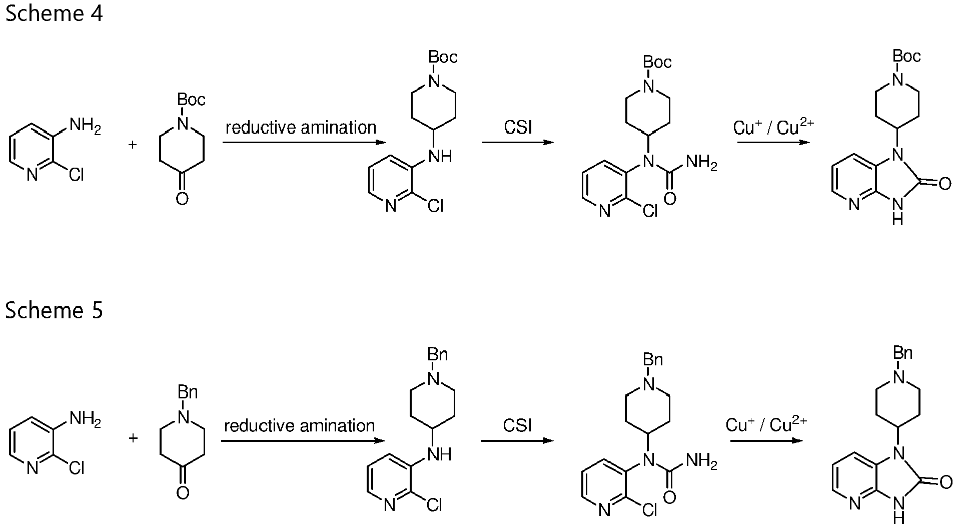

### Synthesis of tert-butyl 4-(1-(2-ch(oropyridin-3yl)ureido)piperidine-1-carboxylate

2-Chloropyridin-3-amine (502 g, 3.92 mol), *tert*-butyl-4-oxopiperidine-1-carboxylate (930 g, 4,67 mol) were stirred in ethyl acetate (7.5 L) at ≤0 5°C, and treated with trifluoroacetic acid (887 g, 7.78 mol), then sodium triacetoxyborohydride (1240 g, 5.85 mol) is added at ≤10°C. The reaction mixture was warmed to room temperature, stirred for 2 h, and quenched with water (1.5 L). The pH was adjusted to 10-11 with sodium hydroxide solution (20%), and the phases were separated. The organic layer was washed with water (3 × 5 L) and concentrated under vacuum to a volume of about 1.5 L. n-Heptane (5 L) was added at 40°C, and the resulting slurry was cooled to ≤5°C and aged for 1 h. The slurry was filtered, the cake was washed with n-heptane (1.5 L) and dried at 50°C under vacuum for 12 h. tert-butyl 4-(2-chloropyridin-3-ylamino)piperidine-1-carboxylate (1170 g, 3.75 mol) was obtained as an off-white solid.
Chlorosulfonyl isocyanate (60.2 g, 0.425 mol) was added to tetrahydrofuran (THF, 300 mL) at room temperature. The mixture was cooled to -10°C. A solution of tert-butyl 4-(2-chloropyridin-3-ylamino)piperidine-1-carboxylate (102.0 g, 0.327 mol) in 1:1 mixture of THF and ethyl acetate (200 mL) is added over a 20 min period. After 10 min at -10°C, water (50 mL) was added dropwise over a 10 min period. The mixture was allowed to warm to room temperature and aged for 30 min. pH is adjusted to 8-9 with sodium hydroxide solution (10%) and the suspension was distilled under reduced pressure to remove THF, the same quantity of ethyl acetate is charged, the residue is filtered and washed with ethyl acetate, and vacuum dried to get the title compound (120 g, 0.338 mol, yield: 96.7%).
Proof of the structure, is done by comparing NMR with literature data, as given in Leahy K. D. et al., Organic Process Research & Development, 2012, Vol. 16, Issue 2, pp 244-249.

### Synthesis of 1-(1-benzylpiperidin-4-yl)-1-(2-chloropyridin--yl)urea

2-Chloropyridin-3-amine (12.8 g, 0.1 mol), 1-benzyl-4-piperidone (25.6 g, 0.12 mol), PTSA (p-toluenesulfonic acid, 0.12 g, 1% wt), and toluene (46 mL) are added into a flask. The mixture was heated to reflux and the generated water was separated out immediately in a Dean-Stark trap. Once no more water was collected, the reaction mixture was cooled down. The resulting imine solution is used in the next step.

Into a flask with MeOH (100 mL) NaBH₄ (38 g, 1 mol) was added in portions at 5-20°C. The imine solution was added dropwise at below 20°C. After the addition was completed, the reaction mixture was stirred at room temperature overnight. 50 g of water was added to quench the reaction mixture. Methanol was distilled, giving a phase separation. The organic phase was concentrated. The residual solid was recrystallized with n-heptane (95 g). After filtration, the wet product was dried at 50°C overnight. N-(1-benzylpiperidin-4-yl)-2-chloropyridin-3-amine (27.4 g, 0.091 mol) is obtained with 91% yield. The HPLC purity was 93.2%.

N-(1-benzylpiperidin-4-yl)-2-chloropyridin-3-amine was further converted into 1-(1-benzyl-piperidin-4-yl)-1-(2-chloropyridin-3-yl)urea following the procedure as described for the conversion of tert-butyl 4-(2-chloropyridin-3-ylamino)piperidine-1-carboxylate *tert-*butyl 4-(1-(2-chloropyridin-3-yl)ureido)piperidine-1-carboxylate.

### Specific examples A-01 to A-12

### Synthesis and isolation of tert-butyl 4-(2-oxo-2,3-dihydro-1H-imidazo[4,5-b]pyridin-1-yl)piperidine-1-carboxylate (R' = Boc)

A flask was charged with 15.25 g of *tert-butyl* 4-(1-(2-chloropyridin-3-yl)ureido)piperidine-1-carboxylate (0.048 mol), 18.5 g of diaminoethane (EDA, 0.308 mol), 13.1 g of potassium carbonate (0.095 mol), 1.1 g, of copper(I)iodide (0.0058 mol), 87 mL of dioxane and 37 mL of water. The mixture is heated to reflux (96°C) and stirred at this temperature for 72 h. The aqueous layer is separated at 90°C. The organic phase is cooled to room temperature (22°C). The suspension is filtered to remove inorganic solids. The mother liquor is concentrated to dryness. The pH of the residue is adjusted to about 6 with diluted HCl.The suspension is filtered off and washed with water. The wet product is dried at 50°C, yielding 11.8 g of the title compound (0.037 mol, corresponding to 77% of the theoretical yield).

Proof of the structure is done by comparison of NMR data with literature data, as given in Leahy K. D. et al., Organic Process Research & Development, 2012, Vol. 16, Issue 2, pp 244-249.

Examples A-02 to A-12 were carried out as Example A-01 - without the final work-up of the product and just analysis of the raw product using HPLC and NMR, with the reagents, amounts and reaction conditions as given in Table 1. The results thereof are also given in Table 1.

### Specific examples B-01 to B-07

### Synthesis and isolation of 1-(1-benzylpiperidin-4-yl)-1H-imidazo[4,5-b]pyridin-2(3H)-one (R' = Bn)

A flask was charged with 10.0 g of 1-(1-benzylpiperidin-4-yl)-1-(2-chloropyridin-3-yl)urea (0.0291 mol), 15.2 g of diaminoethane (EDA, 0.253 mol), 8.0 g of potassium carbonate (0.058 mol), 1.4 g of copper(I) iodide (0.00735 mol), 45 mL of dioxane and 5 mL of water. The resulting mixture was heated to reflux (96°C). The mixture was stirred at this temperature for 72 h. The aqueous layer was separated out at 90°C. The organic phase was cooled to room temperature and analyzed by HPLC. The suspension was filtered off. The mother liquor was concentrated to dryness. The pH of the residue was adjusted to about 6 with diluted HCl.The suspension was filtered off and washed with water. The wet product was dried at 50°C. 8.5 g of the title compound was obtained (0.0278 mol, corresponding to 88% of the theoretical yield).

### Analytics

Hydrogenation of 1-(1-benzylpiperidin-4-yl)-1H-imidazo[4,5-b]pyridin-2(3H)-one to 1H-imidazo[4,5-b]pyridin-2(3H)-one. To proof the structure NMR data is compared with data from Leahy K. D. et al., Organic Process Research & Development, 2012, Vol. 16, Issue 2, pp 244-249.

An autoclave is charged with 0.45 g of 1-(1-benzylpiperidin-4-yl)-1H-imidazo[4,5-b]pyridin-2(3H)-one (1.460 mmol) from example 2, 60 mL of methanol and 0.4 g of palladium on charcoal. The reaction mixture is stirred at 40°C under 35 bar of hydrogen pressure overnight. The resulting solution is concentrated to get an oily solid. The oily solid is dissolved in 10 mL ethanol. 1 mL of 32% hydrochloric acid is added to the precipitate. The suspension is filtered and the wet cake is dried, yielding 0.25 g of 1-(Piperidin-4-yl)-1H-imidazo[4,5-b]pyridin-2(3H)-one dihydrochloride (0.865 mmol). Proof of the structure is done by comparison of NMR data with data from literature, as above.

Examples B-02 to B-07 were carried out as Example B-01- without the final work-up of the product and just analysis of the raw product using HPLC and NMR, with the reagents, amounts and reaction conditions as given in Table 2. The results thereof are also given in Table 2.

### Comparative example C-01

A comparative example following the general process without adding the amino additive led to a yield 0%, as given with the reaction conditions, reagents and amounts thereof in Table 3.

**Table 1: Reaction conditions and results for Examples A-01 to A-12 (R' = Boc)**

| No. | Cu Salt | diamin | solvent | Base | Reaction temp/°C | Reaction time/hrs. | yield |
|---|---|---|---|---|---|---|---|
| A-01 | 0.12 eq. CuI | 6.4 eq. EDA | Dioxane Water 7:3 | 2.0 eq. K₂CO₃ | RF | 72 | 77% |
| A-02 | 0.25 eq. CuI | 2 eq. DMEDA | Dioxane Water 9:1 | 3.0 eq. Na₂CO₃ | RF | 44 | 59% |
| A-03 | 0.25 eq. CuI | 6.4 eq. EDA | Dioxane Water 9:1 | - | RF | 68 | 61% |
| A-04 | 0.20 eq. CuI | 0.25 eq. DMEDA | Sulfolane | 2.0 eq. NaHCO₃ | 130 | 24 | 67% |
| A-05 | 0.25 eq. CuI | 3.2 eq. EDA | Dioxane Water 9:1 | 3.0 eq. K₂CO₃ | RF | 68 | 75% |
| A-06 | 0.48 eq. CuCl | 3.2 eq. EDA | Dioxane Water 9:1 | 3.0 eq. K₂CO₃ | RF | 68 | 77% |
| A-07 | 0.33 eq. CuBr | 3.2 eq. EDA | Dioxane Water 9:1 | 3.0 eq. K₂CO₃ | RF | 68 | 78% |
| A-08 | 0.12 eq. CuI | 6.4 eq. EDA | Dioxane Water 8.7:1.3 | 2.0 eq. K₂CO₃ | RF | 72 | 80% |
| A-09 | 0.25 eq. CuI | 6.4 eq. EDA | Dioxane Water 9:1 | 3eq. K₂CO₃ | RF | 68 | 84% |
| A-10 | 0.25 eq. CuCl | 6 eq. EDA | Toluene Water 9:1 | 3.0 eq. K₂CO₃ | RF | 68 | 97% |
| A-11 | 0.25 eq. CuI | 0.25 eq. DACH | Dioxane | 3.0 eq. K₂CO₃ | 130 | 48 | 85% |
| A-12 | 0.10 eq. CuI | 0.10 eq. DACH | Dioxane | 2.0 eq. K₂CO₃ | 130 | 24 | 54% |

**Table 2: Reaction conditions and results for Examples B-01 to B-07 (R' = Bn)**

| No. | Cu Salt | diamin | solvent | Base | Reaction temp/°C | Reaction time/hrs. | yield |
|---|---|---|---|---|---|---|---|
| B-01 | 0.25 eq. CuI | 8.7 eq. EDA | Dioxane Water 9:1 | 2.0 eq. K₂CO₃ | RF | 72 | 88% |
| B-02 | 0.12 eq. CuI | 6.4 eq. EDA | Dioxane Water 8.7:1.3 | 2.0 eq. K₂CO₃ | RF | 72 | 77% |
| B-03 | 0.50 eq. CuSO₄ | 6 eq. PDA | Dioxane Water 9:1 | 3.0 eq. K₂CO₃ | RF | 60 | 90% |
| B-05 | 0.50 eq. CuSO₄ | 6 eq. EDA | Dioxane Water 9:1 | 3.0 eq. K₂CO₃ | RF | 72 | 93% |
| B-06 | 0.50 eq. CuSO₄ | 6 eq. EDA | Dioxane Water 1:1 | 3.0 eq. K₂CO₃ | RF | 72 | 95% |
| B-07 | 0.50 eq. CuSO₄ | 6 eq. PDA | Dioxane | 3.0 eq. K₂CO₃ | RF | 60 | 86% |

**Table 3: Reaction conditions and results for comparative example C-01, R' = Boc**

| No. | Cu Salt | diamin | solvent | Base | Reaction temp/°C | Reaction time/hrs. | yield |
|---|---|---|---|---|---|---|---|
| C-01 | 0.25 eq. CuI | - | Dioxane Water 9:1 | 2.0 eq. K₂CO₃ | RF | 72 | 0% |

In Tables 1 to 3 the following applies:
- RF:: at reflux temperature
- DMEDA:: N,N'-dimethyl-ethylene diamine
- EDA:: ethylene diamine
- PDA:: propylene diamine
- DACH:: trans-1,2-diaminocyclohexane

## Claims

1. A method of producing a compound of Formula (I) from a compound of Formula (II), comprising:
reacting the compound of Formula (II) in a solvent or a solvent mixture in the presence of a Cu catalyst,
a diamine additive, and
optionally a base, particularly an inorganic base,
to form the compound of Formula (I), 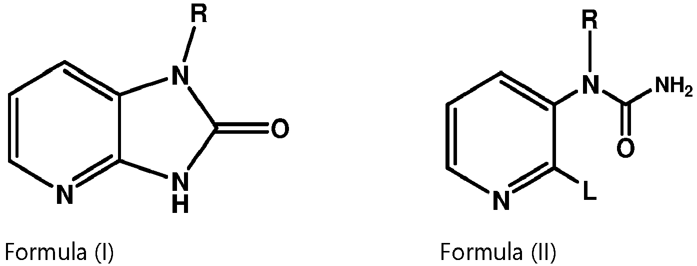 wherein L represents a leaving group, and R represents hydrogen, a substituted or unsubstituted linear, branched and/or cyclic alkyl group that may contain one or more hetero atoms in the linear, branched and/or cyclic alkyl chain, a substituted or unsubstituted aromatic or heteroaromatic group, a substituted or unsubstituted linear, branched and/or cyclic aralkyl or heteroaromatic alkyl group that may contain one or more hetero atoms in the linear, branched and/or cyclic alkanediyl chain, or a substituted or unsubstituted alkylaryl or alkyl heteroaromatic group with at least one linear, branched and/or cyclic alkyl residue that may contain one or more hetero atoms in the linear, branched and/or cyclic alkyl chain.

2. The method according to claim 1, wherein the leaving group L is a halogen.

3. The method according to one of the precedent claims, wherein the leaving group L is chloride.

4. The method according to one of the precedent claims, wherein the Cu catalyst is a copper(I) or a copper(II) salt.

5. The method according to one of the precedent claims, wherein the Cu catalyst is Cul, CuCl, CuBr, Cu(OAc)₂, and/or CuSO₄.

6. The method according to one of the precedent claims, wherein the amount of the copper catalyst is between 0.1 equivalents and 0.6 equivalents related to the compound of Formula (II).

7. The method according to one of the precedent claims, wherein the solvent is an organic solvent or a mixture of organic solvents, containing 0 % to 50 % by weight of water.

8. The method according to one of the precedent claims, wherein the diamine additive is a bidentate ligand.

9. The method according to claim 8, wherein the diamine additive is chosen from the group consisting of N,N'-dimethyl-ethylene diamine (DMEDA), ethylene diamine (EDA), propylene diamine (PDA), or trans-1,2-diaminocyclohexane (DACH), or mixtures thereof.

10. The method according to claim 9, wherein the diamine additive is chosen from EDA and PDA.

11. The method according to one of the precedent claims, wherein the inorganic base is present and is a carbonate or a bicarbonate salt.

12. The method according to one of the precedent claims, wherein the inorganic base is present in an amount of less than 5.0 equivalents in relation to the compound of Formula (II).

13. A method of producing an acid adduct of a compound of Formula (I) from a compound of Formula (II), comprising:
- reacting the compound of Formula (II) in a solvent or a solvent mixture in the presence of
a Cu catalyst,
a diamine additive, and
optionally a base, particularly an inorganic base,
to form the compound of Formula (I), 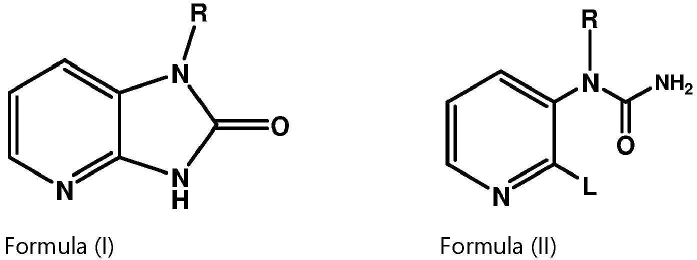 wherein L represents a leaving group, and R represents hydrogen, a substituted or unsubstituted linear, branched and/or cyclic alkyl group that may contain one or more hetero atoms in the linear, branched and/or cyclic alkyl chain, a substituted or unsubstituted aromatic or heteroaromatic group, a substituted or unsubstituted linear, branched and/or cyclic aralkyl or heteroaromatic alkyl group that may contain one or more hetero atoms in the linear, branched and/or cyclic alkanediyl chain, or a substituted or unsubstituted alkylaryl or alkyl heteroaromatic group with at least one linear, branched and/or cyclic alkyl residue that may contain one or more hetero atoms in the linear, branched and/or cyclic alkyl chain, and
- reacting the compound of Formula (I) with an acid.
